# EUROPÄISCHE PATENTSCHRIFT

(11) **EP 3 146 355 B1**
(45) Veröffentlichungstag und Bekanntmachung des Hinweises auf die Patenterteilung: **09.06.2021**
(21) Anmeldenummer: 15732644.8
(22) Anmeldetag: 26.06.2015
(51) Int. Cl.: G01S 5/02, H04W 4/80

(54) **MEDIZINGERÄTESYSTEM UND VERFAHREN ZUR ORTUNG VON MEDIZINGERÄTEN UND MOBILEN STEUERUNGSEINHEITEN DES MEDIZINGERÄTESYSTEMS**
MEDICAL DEVICE SYSTEM AND A METHOD FOR LOCATING MEDICAL DEVICES AND MOBILE CONTROL UNITS OF SAID MEDICAL DEVICE SYSTEM
SYSTÈME D'APPAREILS MÉDICAUX ET PROCÉDÉ POUR LOCALISER DES APPAREILS MÉDICAUX ET DES UNITÉS DE COMMANDE MOBILES DE CE SYSTÈME D'APPAREILS MÉDICAUX

(30) Priorität: 30.06.2014 DE 102014212650
(43) Veröffentlichungstag der Anmeldung: 29.03.2017
(73) Patentinhaber: TRUMPF Medizin Systeme GmbH + Co. KG, 07318 Saalfeld (DE)
(72) Erfinder: MARKA, Rudolf, 85737 Ismaning (DE); ÖZHAN, Serhan, 81379 München (DE); HAN, Gel, 80802 München (DE)
(74) Vertreter: Prüfer & Partner mbB Patentanwälte · Rechtsanwälte
(86) Internationale Anmeldenummer: PCT/EP2015/064541
(87) Internationale Veröffentlichungsnummer: WO 2016/001087

(56) Entgegenhaltungen:
- DE-A1-102010 040 594
- US-A1- 2008 204 322
- US-A1- 2013 053 056
- US-A1- 2013 308 685
- US-A1- 2014 087 752

## Beschreibung

Die Erfindung betrifft ein Medizingerätesystem und ein Verfahren zur Ortung von Medizingeräten und/oder mobilen Steuerungseinheiten des Medizingerätesystems, insbesondere ein System und ein Verfahren, mit denen eine Anwesenheit des Medizingeräts und dessen Steuerungseinheit in einem Raum eines Gebäudebereichs sicher bestimmt werden kann.

Bisher erfolgen Ortungen oder Lokalisierungen von mobilen Steuerungseinheiten von Medizingeräten beispielsweise über Infrarotsignale, die sicherstellen, dass die Steuerungseinheit und das Medizingerät in Sichtkontakt sind. Weitere Möglichkeiten zur Ortung von mobilen Steuerungseinheiten sind die Verwendung eines einzelnen Funkdienstes oder eine Abstandserkennung, beispielsweise über 3D-Sensoren.

Dabei tritt jedoch das Problem auf, dass keine sichere Ortung möglich ist, oder die Position sehr ungenau zu erfassen ist.

Dokument DE 10 2010 040 594 A1 offenbart ein Verfahren zum Orten eines mobilen Telekommunikationsendgeräts, von dem über ein Mobilfunknetz ein Notruf abgesetzt wird. Beim Absetzen des Notrufs sendet eine Notruf-Steuerung über eine im Nahbereich sendende Funk-Schnittstelle, insbesondere eine Bluetooth- und/oder WLAN-Schnittstelle, des mobilen Telekommunikationsendgeräts einen Notfall-Code aus, wobei ein Ortungsgerät den Notfall-Code als solchen erkennt, und wobei das Ortungsgerät die Richtung zu dem den Notfall-Code aussendenden mobilen Telekommunikationsendgerät bestimmt.

In US 2014/0087752 A1 ist eine Bestimmung eines Standorts offenbart, die auf einem Bluetooth-Funkfeuer basiert. An einem mobilen Gerät wird eine Signalabtastung eines Wi-Fi-Signals und eines Bluetooth-Funkfeuers ausgelöst. Ein Standort des mobilen Geräts wird zumindest über mindestens ein von dem mobilen Gerät erfassbares Bluetooth-Funkfeuer bestimmt, wobei das mindestens eine Bluetooth-Funkfeuer von einem ersten Standort ausgesendet wird, und wobei der erste Standort der Standort des mobilen Geräts ist.

Das Dokument US 2013/0308685 A1 zeigt ein drahtloses Kombinationsgerät, das eine Steuerungseinheit enthält, das verwendbare "frequency hopping"-Kanäle für Bluetooth basierend auf Verbindungsqualitätsinformationen eines WLAN-Kanals bestimmt.

Im Dokument US 2013/0053056 A1 ist eine Vorrichtung und ein Verfahren zum Vereinfachen einer Standortbestimmung offenbart. Hierzu kann ein Verfahren ein Identifizieren einer Unbestimmtheit von zumindest einer Abschätzung eines Standorts eines mobilen Geräts enthalten. Signale, die von mehreren Sendern übertragen werden, um die Unbestimmtheit der einen Abschätzung zu reduzieren, können basierend auf mit den Sendern verbundenen Eigenschaften und einem Einschränken der Abschätzung des Standorts des mobilen Geräts in Übereinstimmung mit einem Navigationsweg priorisiert werden.

Dokument US 2008/0204322 A1 offenbart eine Einrichtung zum Bestimmen einer Positionsinformation eines Objekts. Die Einrichtung enthält eine Mehrzahl von empfangenden Elementen und Erfassungsmittel zum Erfassen von durch die empfangenden Elemente empfangenen Signale und zum Erzeugen von entsprechenden Ausgangssignalen. Die Ausgangssignale werden für jedes empfangende Element separat verarbeitet, um jeweilige Parameterwerte zu erhalten, und die Parameterwerte werden verglichen, um Positionsinformationen zu erhalten.

Der Erfindung liegt daher die Aufgabe zugrunde, ein Medizingerätesystem und ein Verfahren zur Ortung von Medizingeräten und mobilen Steuerungseinheiten bereitzustellen, das die obigen Nachteil nicht aufweist, und die eine prozesssichere, genaue Ortung ermöglichen.

Die Aufgabe wird durch ein Medizingerätesystem gemäß Anspruch 1 und ein Verfahren gemäß Anspruch 6 gelöst. Weiterentwicklungen der Erfindung sind Gegenstand der Unteransprüche.

Durch das erfindungsgemäße Medizingerätesystem und Verfahren ist es möglich, mittels zweier unterschiedlicher Erfassungsprinzipien eine genaue, prozesssichere Ortung von Medizingeräten und/oder mobilen Steuerungseinheiten sicherzustellen.

Die Erfindung wird nun anhand von Ausführungsbeispielen unter Bezugnahme auf die beigefügten Zeichnungen erläutert.

Insbesondere zeigen:
- Fig. 1: eine perspektivische Ansicht eines Operationssaals; und
- Fig. 2: einen Grundriss eines Gebäudebereichs mit Operationssälen.

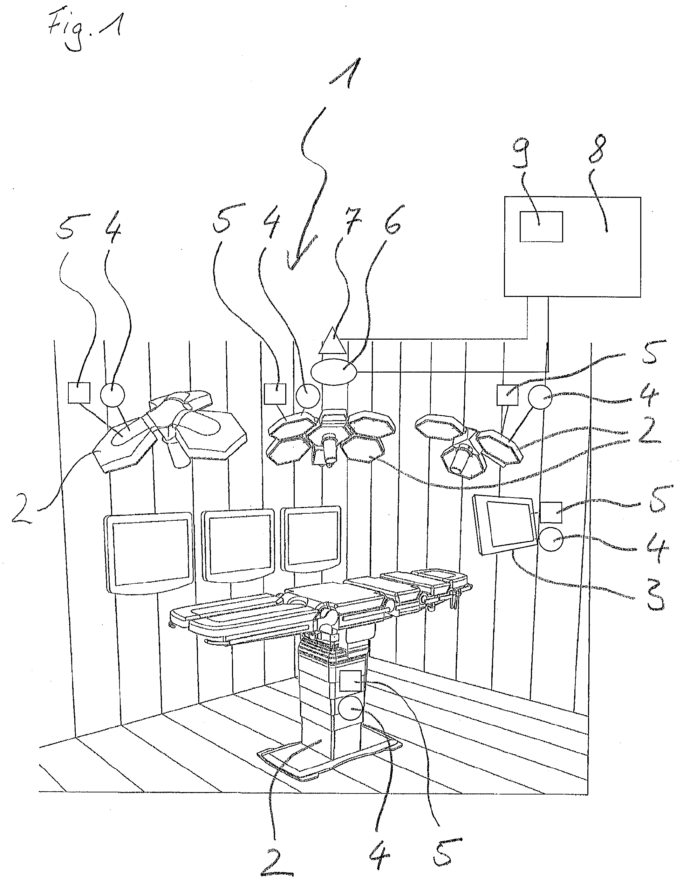

In Fig. 1 ist eine perspektivische Ansicht eines Operationssaals als Raum 1 in einem Gebäudebereich, beispielsweise in einem Krankenhaus gezeigt. In dem Raum 1 sind ein Operationstisch und mehrere Operationsleuchten als Medizingeräte 2 dargestellt. Ferner ist optional ein Tablet-PC als eine mobile Steuerungseinheit 3 zum Steuern von zumindest einem der Medizingeräte 2 vorgesehen, wobei dann ein Medizingerätesteuerungssystem gebildet ist. Die Medizingeräte 2 und die mobile Steuerungseinheit 3 sind in Abhängigkeit davon, ob die Medizingeräte 2 und/oder die mobile Steuerungseinheit 3 geortet werden sollen, jeweils optional mit einem WLAN-Sendemodul 4 und einem weiteren Funk-Sendemodul 5 ausgestattet.

In dem Raum 1 sind ferner ein WLAN-Access-Point 6 und ein weiteres Funk-Lesemodul 7 vorhanden. Sofern mehrere solche Räume 1 vorhanden sind, ist jeder Raum mit einem WLAN-Access-Point 6 und einem weiteren Funk-Lesemodul 7 versehen. In einem Fall, in dem eine Signalübertragung durch Wände der Räume 1 möglich ist, ist alternativ ein einziger WLAN-Access-Point 6 und ein einziges weiteres Funk-Lesemodul 7 für mehrere Räume vorgesehen. Der WLAN-Access-Point 6 und das weitere Funk-Lesemodul 7 sind mit einer Steuerungsvorrichtung 8 verbunden.

Diese Komponenten, die WLAN-Sendemodule 4, die weiteren Funk-Sendemodule 5, die WLAN-Access-Points 6, die weiteren Funk-Lesemodule 7 und die Steuerungsvorrichtung 8, sind Bestandteile eines Medizingerätesystems.

In diesem Ausführungsbeispiel sind eine einzelne mobile Steuerungseinheit 3 und vier Medizingeräte 2, nämlich der Operationstisch und drei Operationsleuchten dargestellt. In alternativen Ausführungsformen können auch mehrere mobile Steuerungseinheiten 3 und/oder eine andere Anzahl von Medizingeräten 2 vorgesehen sein.

Der WLAN-Access-Point 6 ist mit mindestens einer Antenne, insbesondere einer Smart-Antenne, versehen. Die Smart-Antenne kann die Richtung bestimmen, aus der eine Anfrage eines Gerätes über WLAN kommt und sendet oder empfängt daraufhin gezielt Daten aus dieser Richtung oder in diese Richtung. Dadurch gibt es beim Einsatz von vielen Geräten weniger Überlappungen und Störungen, und es ist ein höherer Datendurchsatz möglich. Der WLAN-Access-Point 6 arbeitet in einem 5 GHz-Band. Alternativ arbeitet er in zwei verschiedenen Frequenzbändern, nämlich dem 2,4 GHz-Band und dem 5 GHz-Band oder in einer Kombination aus dem 2,4 GHz-Band und dem 5 GHz-Band, um eventuelle Störungen in einem bestimmten Band zu vermeiden.

Die WLAN-Sendemodule 4 der Medizingeräte 2 und der mobilen Steuerungseinheit 3 sind mit einer jeweiligen Adresse versehen und senden Signale in dem korrespondierenden Frequenzband des WLAN-Access-Points 6 aus.

Mit dem WLAN-Access-Point 6 wird eine Signalstärke der von den WLAN-Sendemodulen 4 ausgesendeten Signale erfasst und über die empfangene Adresse dem jeweiligen WLAN-Sendemodul 4 zugeordnet.

Die weiteren Sendemodule 5 der Medizingeräte 2 und der mobilen Steuerungseinheit 3 sowie die weiteren Funk-Lesemodule 7 sind in diesem Ausführungsbeispiel "Bluetooth Low Energy" (BLE) -Module. In einer alternativen Ausführungsform sind die Funk-Sendemodule und -Lesemodule beispielsweise RFID-Module.

Die Steuerungsvorrichtung 8 ist mit einem Speicherbereich 9 versehen, in dem Datensätze von später erläuterten Signalstärkekombinationen, sogenannten "Fingerprints", gespeichert werden können.

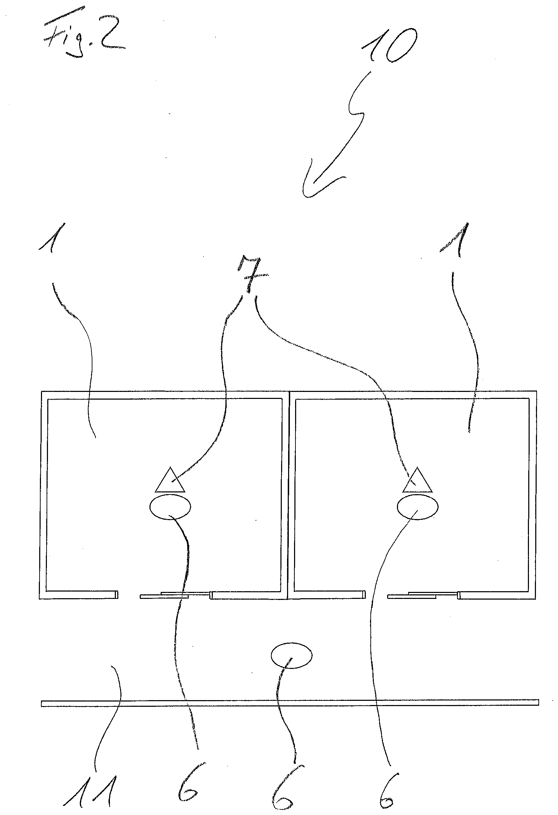

In Fig. 2 ist eine Karte von einem Grundriss eines Gebäudebereichs 10 gezeigt. In dem Gebäudebereich 10 sind zwei Räume 1, hier die Operationssäle, dargestellt. Außerhalb der Räume erstreckt sich ein Flur 11.

In Fig. 2 ist zu sehen, dass sich in jedem der Räume 1 einer der WLAN-Access-Points 6 befindet. Auch auf dem Flur 11 ist einer der WLAN-Access-Points 6 vorgesehen. In den Räumen 1 ist ferner jeweils eines der weiteren Funk-Lesemodule 7 angeordnet.

Im Betrieb wird eine Ortung der Medizingeräte 2 und der mobilen Steuerungseinheit 3 über die Erfassung der Signalstärken durch die WLAN-Einrichtung und durch die weitere Funkeinrichtung durchgeführt.

Für die Erfassung durch die WLAN-Einrichtung werden in dem Speicherbereich 9 der Steuerungsvorrichtung 8 im Voraus Datensätze für einzelne Positionen gespeichert. Dazu werden, beispielsweise mit einem Laptop mit einer zusätzlichen omni-direktionalen Antenne, die jeweiligen Signalstärken an den einzelnen WLAN-Access-Points 6 für die einzelnen Positionen einmalig die jeweiligen Datensätze ("Fingerprints") in dem Speicherbereich 9 abgelegt.

Ferner wird in dem Speicherbereich 9 auch ein Bild einer Karte mit dem Grundriss des zu erfassenden Gebäudebereichs gespeichert.

Die Signalstärken der von den WLAN-Sendemodulen 4 ausgesendeten Signale werden, über die jeweilige Adresse den Medizingeräten 2 oder der mobilen Steuerungseinheit 3 zugeordnet, über die drei WLAN-Access-Points 6 erfasst. Alternativ ist auch die Erfassung über mehr oder weniger WLAN-Access-Points 6 möglich.

Über einen ausgewählten Algorithmus werden dann die Signalstärken mit den "Fingerprints" verglichen und anhand der Karte berechnet, d.h. ermittelt, in welchem der Räume 1 sich das Medizingerät 2 und/oder die mobile Steuerungseinheit 3 befinden. Der Raum 1, in dem sich die mobile Steuerungseinheit 3 befindet, ist dann bekannt, wenn sich die aktuell empfangenen Signalstärken der mindestens drei Access Points 6 im Speicherbereich 9 in derselben oder annähernd derselben Kombination befinden. Darüber kann auf die Position in X und Y, an der schon einmal beim Aufnehmen der Fingerprints die (annähernd) gleiche Kombination gemessen wurde, rückgeschlossen werden.

Die Ortung über BLE erfolgt über die mittels der weiteren Funk-Lesemodule 7 erfassten Signalstärken der von den weiteren Funk-Sendemodule 7 ausgesendeten Signale. In den Räumen 1 ist jeweils ein BLE-Lese-Modul 7 an der Decke installiert. Das BLE-Lese-Modul 7 muss nicht zwingend an der Decke montiert sein, sollte sich aber zentral im Raum befinden. Die Empfangsempfindlichkeit der BLE-Lese-Module 7 wird so verringert, dass eine klare Unterscheidung der erfassten Signalstärken aus den unterschiedlichen Räumen möglich ist. Die Empfangsempfindlichkeit sollte so eingestellt sein, dass Signale von außerhalb des Raums 1, in dem sich das jeweilige BLE-Lese-Modul 7 befindet, nicht oder möglichst mit minimaler Signalstärke erfasst werden. Anhand einer festgelegten Mindestsignalstärke wird dann bestimmt, in welchem Raum sich das Medizingerät 2 oder die mobile Steuerungseinheit 3 befindet. Jedem Raum ist in diesem Ausführungsbeispiel genau ein BLE-Lese-Modul 7 zugeordnet. In alternativen Ausführungsbeispielen können jedem Raum auch mehrere oder kein BLE-Lese-Modul 7 zugeordnet sein. Die Bestimmung, in welchem Raum 1 sich das Medizingerät 2 oder die mobile Steuerungseinheit 3 befindet, wird durch die Steuerungsvorrichtung 8 ausgeführt.

Sobald die mobile Steuerungseinheit 3 oder eines der Medizingeräte 2 eingeschaltet wird, werden durch das WLAN-Sendemodul 4 Signale ausgesendet, deren Signalstärken an den drei WLAN-Access-Points 6 erfasst und zur Bestimmung der Position an die Steuerungsvorrichtung 8 geschickt. Gleichzeitig werden durch das weitere Funk-Sendemodul 5 Signale ausgesendet und die BLE-Lese-Module 7 empfangen das Signal des weiteren Funk-Sendemoduls 5. Optional werden bei den Medizingeräten 2 WLAN- und Bluetooth-Signale ständig geschickt, wohingegen in der mobilen Steuerungseinheit 3 Bluetooth-Signale immer geschickt werden und WLAN-Signale nur, wenn die mobile Steuerungseinheit 3 eingeschaltet ist. In alternativen Ausführungsformen werden die Signale des WLAN-Sendemoduls 4 und des weiteren Funk-Sendemoduls 5 nicht gleichzeitig sondern beispielsweise sequenziell, ggf. zyklisch, ausgestrahlt und/oder empfangen. Die Bestimmungen der aktuellen Position über die WLAN-Signalstärken und des aktuellen Raums über die BLE-Lokalisierung werden überlagert und als ein Ergebnis wird eine eindeutige Bestimmung des Raums 1, in dem sich das Medizingerät 2 oder die mobile Steuerungseinheit befindet, ermöglicht.

Die einzelnen Ausführungsbeispiele sind miteinander kombinierbar.

## Patentansprüche

1. Medizingerätesteuerungssystem mit einem Medizingerätesystem, wobei das Medizingerätesystem aufweist:
mindestens ein Medizingerät (2) mit einem WLAN-Sendemodul (4), und
mindestens eine mobile Steuerungseinheit (3) für ein Medizingerät (2), mit einem WLAN-Sendemodul (4),
mindestens drei WLAN-Access-Points (6) mit jeweils mindestens einer Antenne,
mindestens ein weiteres Funk-Lesemodul (7), und
eine mit einem von den mindestens drei WLAN-Access-Points (6) und dem mindestens einen weiteren Funk-Lesemodul (7) verbundene Steuerungsvorrichtung (8), wobei
das mindestens eine Medizingerät (2) mittels der mobilen Steuerungseinheit (3) ansteuerbar ist
die WLAN-Access-Points (6) ausgebildet sind, eine Stärke eines Signals des mindestens einen WLAN-Sendemoduls (4) zu erfassen,
die Steuerungsvorrichtung (8) einen Speicherbereich (9) für Datensätze von Signalstärkekombinationen für einzelne Positionen des mindestens einen Medizingeräts (2) und der mindestens einen mobilen Steuerungseinheit (3) aufweist,
die Steuerungsvorrichtung (8) einen Speicherbereich (9) für Datensätze einer Karte aufweist, die einen Grundriss eines Bereichs enthält, in dem die Signalstärkekombinationen erfasst werden,
der erfasste Bereich Räume (1) in einem Gebäudebereich (10) und einen außerhalb der Räume (1) liegenden Bereich (11) aufweist, und in den Räumen (1) jeweils ein einzelner WLAN-Access-Point (6) und in dem außerhalb des Raums (1) liegenden Bereich (11) ein einzelner WLAN-Access-Point (6) vorgesehen sind, und die Steuerungsvorrichtung (8) ausgebildet ist, anhand der abgespeicherten Datensätze zu bestimmen, ob sich eine derzeitig erfasste Position des mindestens einen Medizingeräts (2) und der mindestens einen mobilen Steuerungseinheit (3) innerhalb oder außerhalb eines der Räume (1) und in welchem Raum (1) befindet,
so dass eine Anwesenheit des Medizingeräts (2) und dessen Steuerungseinheit (3) in einem Raum des Gebäudebereichs sicher bestimmt werden kann,
wobei das mindestens eine Medizingerät (2) ein weiteres Funk-Sendemodul (5) und
die mindestens eine mobile Steuerungseinheit (3) für ein Medizingerät (2) ein weiteres Funk-Sendemodul (5) aufweist, und
das mindestens eine weitere Funk-Lesemodul (7) ausgebildet ist, eine Stärke eines Signals des mindestens einen weiteren Funk-Sendemoduls (5) zu erfassen.

2. Medizingerätesteuerungssystem gemäß Anspruch 1, wobei der mindestens eine WLAN-Access-Point (6) Sende- und Empfangseinrichtungen für unterschiedliche Frequenzen aufweist.

3. Medizingerätesteuerungssystem gemäß Anspruch 2, wobei in einem Raum (1) ein einzelnes weiteres Funk-Lesemodul (7) vorgesehen ist, und wobei das Medizingerätesystem ausgebildet ist, über die Stärke des Signals zu bestimmen, ob sich das mindestens eine weitere Funk-Sendemodul (5) innerhalb oder außerhalb des Raums (1) befindet.

4. Medizingerätesteuerungssystem gemäß einem der vorangehenden Ansprüche, wobei das weitere Funk-Sendemodul (5) ein Bluetooth-Low-Energy-Sendemodul ist und das weitere Funk-Lesemodul (7) ein Bluetooth-Low-Energy-Lesemodul ist.

5. Medizingerätesteuerungssystem gemäß einem der Ansprüche 1 bis 4, wobei das weitere Funk-Sendemodul (5) und das weitere Funk-Lesemodul (7) RFID-Module sind.

6. Verfahren zur Ortung von Medizingeräten (2) und mobilen Steuerungseinheiten (3) mit einem Medizingerätesteuerungssystem gemäß einem der Ansprüche 1 bis 5, wobei
die Signalstärke des erfassten Signals des mindestens einen WLAN-Sendemoduls (4) über den WLAN-Access-Point (6) bestimmt wird,
die Signalstärke des erfassten Signals des mindestens einen weiteren Funk-Sendemoduls (5) über das weitere Funk-Lesemodul (7) bestimmt wird, und
eine Position des mindestens einen Medizingeräts (2) und der mindestens einen mobilen Steuerungseinheit (3) anhand der bestimmten Signalstärken des mindestens einen WLAN-Sendemoduls (4) und des mindestens einen weiteren Funk-Sendemoduls (5) bestimmt wird,
so dass eine Anwesenheit des Medizingeräts (2) und dessen Steuerungseinheit (3) in einem Raum des Gebäudebereichs sicher bestimmt werden kann.

7. Verfahren gemäß Anspruch 6, wobei die Bestimmung der Signalstärke des WLAN-Sendemoduls (4) und des weiteren Funk-Sendemoduls (5) gleichzeitig erfolgt.

8. Verfahren gemäß Anspruch 6 oder 7, wobei die Bestimmung der Position mittels mehrerer WLAN-Access-Points (6) und mittels eines einem Raum (1) eines Gebäudebereichs (10) zugeordneten weiteren Funk-Lesemoduls (7) erfolgt.

9. Verfahren gemäß einem der Ansprüche 6 bis 8, wobei, wenn bestimmt wird, dass die mobile Steuerungseinheit (3) nicht in demselben Raum (1) eines Gebäudebereichs (10) mit einem anzusteuernden Medizingerät (2) ist, eine Ansteuerungsfunktion der mobilen Steuerungseinheit (3) für das anzusteuernde Medizingerät (2) deaktiviert wird.

10. Verfahren gemäß einem der Ansprüche 6 bis 9, wobei die Signalstärkekombinationen für die einzelnen Positionen im Voraus mittels einer Erfassungseinrichtung bestimmt werden.

## Claims

1. Medical device control system having a medical device system, wherein the medical device system comprises:
at least one medical device (2) having a WLAN transmitting module (4) and
at least one mobile control unit (3) for a medical device (2), having a WLAN transmitting module (4),
at least three WLAN access points (6) respectively having at least one antenna,
at least one further radio reading module (7), and
a control device (8) joined to one of the at least three WLAN access points (6) and to the at least one further radio reading module (7),
wherein
the at least one medical device (2) can be controlled by means of the mobile control unit (3),
the WLAN access points (6) are configured to detect a strength of a signal of the at least one WLAN transmitting module (4),
the control device (8) comprises a memory area (9) for datasets of signal strength combinations for individual positions of the at least one medical device (2) and the at least one mobile control unit (3),
the control device (8) comprises a memory area (9) for data sets of a map including a top view of an area in which the signal strength combinations are detected,
the detected area comprises rooms (1) in a building area (10) and an area (11) outside the rooms (1), and one single WLAN access point (6) is respectively provided in the rooms (1) and a single WLAN access point (6) is provided in the area (11) outside the room (1), and the control device (8) is configured to determine by means of the stored data sets whether a currently detected position of the at least one medical device (2) and of the at least one mobile control unit (3) is inside or outside of one of the rooms (1) and in which room (1),
so that a presence of the medical device (2) and of its control unit (3) in a room of the building area can reliably be determined,
wherein the at least one medical device (2) comprises a further radio transmitting module (5) and
the at least one mobile control unit (3) for a medical device (2) comprises a further radio transmitting module (5), and
the at least one further radio reading module (7) is configured to detect a strength of a signal of the at least one further radio transmitting module (5).

2. Medical device control system according to claim 1, wherein the at least one WLAN access point (6) comprises transmitting and receiving devices for different frequencies.

3. Medical device control system according to claim 2, wherein a single further radio reading module (7) is provided in one room (1), and wherein the medical device system is configured to determine via the strength of the signal whether the at least one further radio transmitting module (5) is located within or outside the room (1).

4. Medical device control system according to anyone of the preceding claims, wherein the further radio transmitting module (5) is a Bluetooth Low Energy transmitting module and the further radio reading module (7) is a Bluetooth Low Energy reading module.

5. Medical device control system according to anyone of the claims 1 to 4, wherein the further radio transmitting module (5) and the further radio reading module (7) are RFID modules.

6. Method for locating medical devices (2) and mobile control units (3) by a medical device control system according to anyone of claims 1 to 5, wherein
the signal strength of the detected signal of the at least one WLAN transmitting module (4) is determined via the WLAN access point (6),
the signal strength of the detected signal of the at least one further radio transmitting module (5) is determined via the further radio reading module (7), and
a position of the at least one medical device (2) and of the at least one mobile control unit (3) is determined by means of the determined signal strengths of the at least one WLAN transmitting module (4) and of the at least one further radio transmitting module (5),
so that a presence of the medical device (2) and its control unit (3) in one room of the building area can reliably be determined.

7. Method according to claim 6, wherein the determination of the signal strength of the WLAN transmitting module (4) and of the further radio transmitting module (5) is performed simultaneously.

8. Method according to claim 6 or 7, wherein the determination of the position is performed by means of several WLAN access points (6) and by means of a further radio reading module (7) assigned to a room (1) of a building area (10).

9. Method according to anyone of the claims 6 to 8, wherein a control function of the mobile control unit (3) for the medical device (2) to be controlled is deactivated when it is determined that the mobile control unit (3) is not located in the same room (1) of a building area (10) with a medical device (2) to be controlled.

10. Method according to anyone of the claims 6 to 9, wherein the signal strength combinations for the individual positions are determined by means of a detection device in advance.

## Revendications

1. Système de commande d'appareils médicaux avec un système d'appareils médicaux, où le système d'appareils médicaux présente :
au moins un appareil médical (2) avec un module d'émission WLAN (4), et
au moins une unité de commande mobile (3) pour un appareil médical (2), avec un module d'émission WLAN (4),
au moins trois points d'accès WLAN (6) chacun avec au moins une antenne,
au moins un autre module de lecture radio (7), et
un dispositif de commande (8) relié à l'un des au moins trois points d'accès WLAN (6) et au au moins un autre module de lecture radio (7), où
le au moins un appareil médical (2) peut être commandé au moyen de l'unité de commande mobile (3),
les points d'accès WLAN (6) sont conçus pour détecter une intensité d'un signal du au moins un module d'émission WLAN (4),
le dispositif de commande (8) présente une zone de mémoire (9) pour des ensembles de données de combinaisons d'intensité de signal pour des positions individuelles du au moins un appareil médical (2) et de la au moins une unité de commande mobile (3),
le dispositif de commande (8) présente une zone de mémoire (9) pour des ensembles de données d'une carte qui contient un plan d'une zone dans laquelle les combinaisons d'intensité de signal sont détectées,
la zone détectée présente des pièces (1) dans une zone de bâtiment (10) et une zone (11) située à l'extérieur des pièces (1), et dans chacune des pièces (1) un point d'accès WLAN (6) unique et dans la zone (11) située à l'extérieur de la pièce (1) un point d'accès WLAN individuel (6) sont prévus, et le dispositif de commande (8) est conçu pour déterminer, à l'aide des ensembles de données mémorisés, si une position détectée actuellement du au moins un appareil médical (2) et de la au moins une unité de commande mobile (3) se trouve à l'intérieur ou à l'extérieur de l'une des pièces (1) et dans quelle pièce (1),
de sorte qu'une présence de l'appareil médical (2) et de son unité de commande (3) dans une pièce de la zone de bâtiment peut être déterminée de manière fiable,
où le au moins un appareil médical (2) présente un autre module d'émission radio (5) et
la au moins une unité de commande mobile (3) pour un appareil médical (2) présente un autre module d'émission radio (5), et
le au moins un autre module de lecture radio (7) est conçu pour détecter une intensité d'un signal du moins un autre module d'émission radio (5).

2. Système de commande d'appareils médicaux selon la revendication 1, où le au moins un point d'accès WLAN (6) présente des organes d'émission et de réception pour des fréquences différentes.

3. Système de commande d'appareils médicaux selon la revendication 2, où un seul autre module de lecture radio (7) est prévu dans une pièce (1), et où le système d'appareils médicaux est conçu pour déterminer, par le biais de l'intensité du signal, si le au moins un autre module d'émission radio (5) se trouve à l'intérieur ou à l'extérieur de la pièce (1).

4. Système de commande d'appareils médicaux selon l'une des revendications précédentes, où l'autre module d'émission radio (5) est un module d'émission basse énergie Bluetooth et l'autre module de lecture radio (7) est un module de lecture basse énergie Bluetooth.

5. Système de commande d'appareils médicaux selon l'une des revendications 1 à 4, où l'autre module d'émission radio (5) et l'autre module de lecture radio (7) sont des modules RFID.

6. Procédé de localisation d'appareils médicaux (2) et d'unités de commande mobiles (3) avec un système de commande d'appareils médicaux selon l'une des revendications 1 à 5, où
l'intensité de signal du signal détecté du au moins un module d'émission WLAN (4) est déterminée par le biais du point d'accès WLAN (6),
l'intensité de signal du signal détecté du au moins un autre module d'émission radio (5) est déterminée par le biais de l'autre module de lecture radio (7), et
une position du au moins un appareil médical (2) et de la au moins une unité de commande mobile (3) est déterminée à l'aide des intensités de signal déterminées du au moins un module d'émission WLAN (4) et du au moins un autre module d'émission radio (5),
de sorte qu'une présence de l'appareil médical (2) et de son unité de commande (3) dans une pièce de la zone de bâtiment peut être déterminée de manière fiable.

7. Procédé selon la revendication 6, où la détermination de l'intensité de signal du module d'émission WLAN (4) et de l'autre module d'émission radio (5) a lieu simultanément.

8. Procédé selon la revendication 6 ou 7, où la détermination de la position a lieu au moyen de plusieurs points d'accès WLAN (6) et au moyen d'un autre module de lecture radio (7) affecté à une pièce (1) d'une zone de bâtiment (10).

9. Procédé selon l'une des revendications 6 à 8, où, quand il est déterminé que l'unité de commande mobile (3) n'est pas dans la même pièce (1) d'une zone de bâtiment (10) avec un appareil médical (2) à commander, une fonction de commande de l'unité de commande mobile (3) est désactivée pour l'appareil médical (2) à commander.

10. Procédé selon l'une des revendications 6 à 9, où les combinaisons d'intensité de signal pour les positions individuelles sont déterminées à l'avance au moyen d'un dispositif de détection.
